# EUROPEAN PATENT SPECIFICATION

(11) **EP 4 343 431 B1**
(45) Date of publication and mention of the grant of the patent: **09.09.2026**
(21) Application number: 23199389.0
(22) Date of filing: 25.09.2023
(51) Int. Cl.: G03B 42/02, G03C 3/00, A61B 6/42, A61B 6/51, G03B 42/04, A61B 6/00

(54) **IMAGING PLATE PROTECTION ASSEMBLY AND MEDICAL IMAGING ASSEMBLY**
BILDGEBUNGSPLATTENSCHUTZANORDNUNG UND MEDIZINISCHE BILDGEBUNGSANORDNUNG
ENSEMBLE DE PROTECTION DE PLAQUE D'IMAGERIE ET ENSEMBLE D'IMAGERIE MÉDICALE

(30) Priority: 26.09.2022 JP 2022152377
(43) Date of publication of application: 27.03.2024
(73) Proprietor: J. MORITA MFG. CORP., Fushimi-ku Kyoto-shi Kyoto 612-8533 (JP)
(72) Inventor: KIRIMURA, Susumu, Kyoto, 612-8533 (JP); SUGIHARA, Yoshito, Kyoto, 612-8533 (JP); FUJII, Yusuke, Kyoto, 612-8533 (JP); HIROTA, Masayuki, Kyoto, 612-8533 (JP); YOSHIKAWA, Hideki, Kyoto, 612-8533 (JP)
(74) Representative: Müller Hoffmann & Partner

(56) References cited:
- US-A- 5 044 008
- US-A- 5 289 522
- US-A- 6 062 730
- US-A1- 2005 259 791
- US-A1- 2011 235 874

## Description

### Field of the Invention

The present disclosure relates to an assembly capable of accommodating a plurality of imaging plates (IP).

### Description of the Background Art

Japanese Patent Application Laid-Open No. 2011-212401 describes an assembly capable of accommodating two imaging plates. The assembly described in Japanese Patent Application Laid-Open No. 2011-212401 includes a container configured to receive and hold a first imaging plate and a second imaging plate in an overlapping direction, and a bag having a fluid impervious seal structure configured to receive and hold the first imaging plate, the second imaging plate, and the container.

In the assembly of Japanese Patent Application Laid-Open No. 2011-212401, since the plurality of imaging plates held in the container are accommodated in one bag, it is necessary to prepare a plurality of types of bags having different sizes according to the number of imaging plates to be used. In addition, since the bag covers the container holding the plurality of imaging plates, the thickness of the contents in the bag is large, and the mouth of the bag is hardly closed. As a result, body fluid such as saliva easily enters the inside of the bag.

US 2011/235874 A1, US 2005/259791 A1, US 6 062 730 A, and US 5 289 522 A each disclose assemblies for imaging plates.

### SUMMARY

An object of the present disclosure is to provide a technique of an assembly in which a plurality of imaging plates can be accommodated, the technique reducing the number of types of sizes of a waterproof bag and making it difficult for body fluid such as saliva to enter the inside of a bag covering the imaging plate.

This object is achieved by the subject-matter of independent claims 1 and 5.

Because the imaging plate protection assembly includes the plurality of waterproof bags accommodating one-to-one the plurality of imaging plates, waterproof bags having the same size can be used regardless of the number of the plurality of imaging plates held by the IP holding member. Accordingly, the number of types of sizes of the waterproof back can be reduced. Furthermore, the thickness of the contents in the waterproof bag can be reduced as compared with the case where the waterproof bag accommodates the entire IP holding member holding the plurality of imaging plates. As a result, the waterproof bag is easily closed, and body fluid such as saliva is less likely to enter the waterproof bag.

These and other objects, features, aspects and advantages of the present invention will become more apparent from the following detailed description of the present invention when taken in conjunction with the accompanying drawings.

### BRIEF DESCRIPTION OF THE DRAWINGS

FIGS. 1 to 11 are schematic views each illustrating an example of a medical imaging assembly;
FIG. 12 is a schematic view illustrating an example of an IP holding member; and
FIGS. 13 to 16 are schematic views each illustrating an example of the medical imaging assembly.

### DETAILED DESCRIPTION

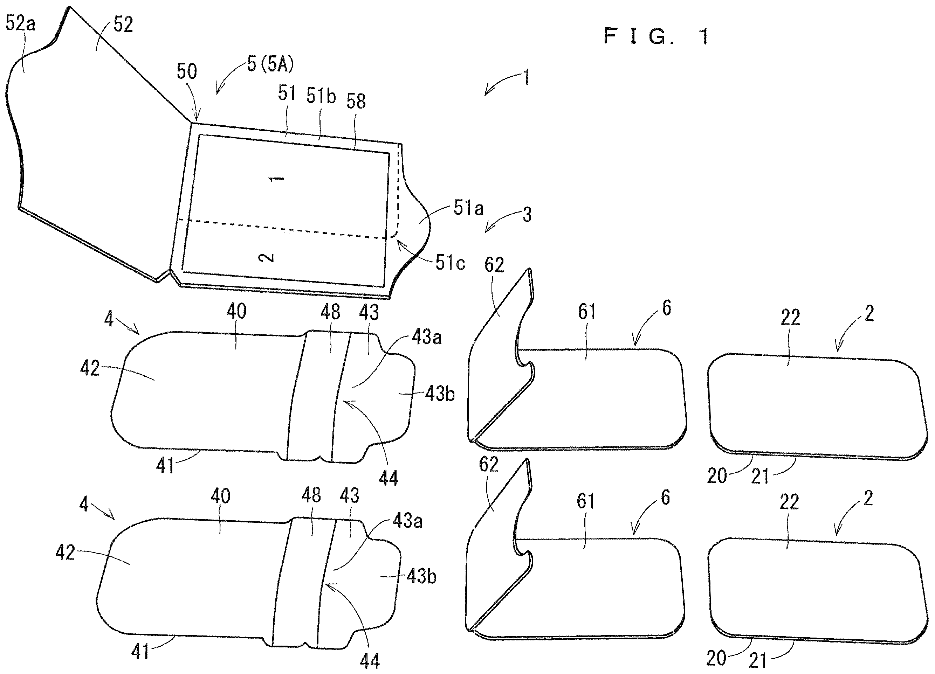

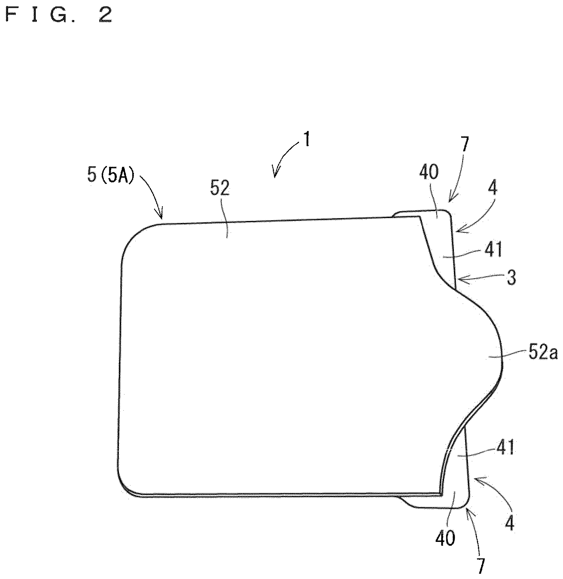

FIGS. 1 and 2 are schematic views illustrating an example of a medical imaging assembly 1 (also referred to as an IP assembly 1) including a plurality of imaging plates 2. FIG. 1 illustrates an example of the IP assembly 1 before assembly, and FIG. 2 illustrates an example of the IP assembly 1 after assembly. The IP assembly 1 is used, for example, for photographing an oral cavity of a person. The IP assembly 1 is used, for example, in occlusal photography. The application of the IP assembly 1 is not limited thereto.

As illustrated in FIGS. 1 and 2, the IP assembly 1 includes, for example, the plurality of imaging plates 2 and an imaging plate protection assembly 3 capable of accommodating the plurality of imaging plates 2. In the example of FIGS. 1 and 2, the number of imaging plates 2 is two, but the number of imaging plates 2 may be three or more. Note that the IP assembly 1 can also be used in a state of accommodating a single imaging plate.

The imaging plate protection assembly 3 (also referred to as a protection assembly 3) includes, for example, a plurality of waterproof bags 4 that are configured to accommodate one-to-one a plurality of imaging plates 2, and an IP holding member 5 that is configured to hold the plurality of imaging plates 2 accommodated in the plurality of waterproof bags 4 at predetermined positions. In addition, the protection assembly 3 includes, for example, a plurality of cover members 6 covering one-to-one the plurality of imaging plates 2. The waterproof bag 4 accommodates the imaging plate 2 covered with the cover member 6. The waterproof bag 4 corresponds to a bag having a fluid impervious seal structure disclosed in Japanese Patent Application Laid-Open No. 2011-212401 described above. The protection assembly 3 may not include the cover members 6. In a configuration in which the protection assembly 3 does not include the cover members 6, the IP holding member 5 (in an IP holding member 5A, in particular, the fixed portion 51 and a cover portion 52 (described later), and in an IP holding member 5B, in particular, main wall portions 501 and 502) may also serve to reduce mechanical damage of the imaging plate 2.

The imaging plate 2 has a flat shape including a radiograph formation layer 20, and is a recording medium for recording a radiograph. The imaging plate 2 has a front surface (in other words, a surface on a front side) on which the radiograph formation layer 20 is located and a back surface opposite to the front surface. The imaging plate 2 has, for example, a substantially rectangular flat shape with rounded four corners. The radiograph formation layer 20 is a layer that accumulates energy of the irradiated radiation and emits photostimulated light according to the accumulated energy. The imaging plate 2 has a film formed of resin. The film has a front surface (in other words, a surface on a front side) on which the radiograph formation layer 20 is provided and a back surface opposite to the front surface. The radiograph formation layer 20 is made of, for example, a photostimulable phosphor applied to the surface of the film. As the radiation radiated to the radiograph formation layer 20, for example, an X-ray is adopted. When the X-ray from the X-ray generator is transmitted through a photographing object and radiated to the imaging plate 2, energy corresponding to the intensity of the X-ray is accumulated in the radiograph formation layer 20. Since the intensity of the X-ray is based on the distribution of the X-ray absorption region in the photographing object, the distribution of the energy accumulated in the radiograph formation layer 20 is a radiograph of the photographing object by the X-ray. In this manner, the imaging plate 2 records, for example, the radiograph by the X-ray as a latent image.

FIG. 2 illustrates an example of a state in which the plurality of imaging plates 2 accommodated one-to-one in the plurality of waterproof bags 4, are held by the IP holding member 5. The **IP** assembly 1 is arranged, for example, in an oral cavity of a person in the state of FIG. 2. For example, there are a plurality of types of sizes of the imaging plates 2. The type and number of imaging plates to be used are appropriately selected according to a photographing object.

In dental X-ray photography using an imaging plate, generally, an imaging plate having a small size tends to be more frequently used than an imaging plate having a large size. This tendency is due to the background that scanning a large-sized imaging plate using a general detector conventionally used in the field of dental X-ray photography has many problems and high technical hurdles. On the contrary, in a case where a detector capable of scanning an imaging plate having a large size is mounted, the cost of the apparatus itself increases, or the apparatus is overperformance in the detection of an imaging plate (having a small size) that is generally used. Therefore, if the photography can be performed using the imaging plate having a small size as in the case of using the imaging plate having a large size, various types of dental X-ray photography can be performed even with the conventional apparatus, and the application is expanded. Note that the imaging plate having a small size and the imaging plate having a large size described herein are expressions for convenience of indicating separation between a size that can be read in normal scanning by a conventionally used general detector and a size that cannot be read. For example, the following is assumed. The imaging plate having a small size is, specifically, in dental X-ray photography, an imaging plate called "size 0" generally used for standard photography of children, an imaging plate called "size 1" generally used for bite wing method photography of children, an imaging plate called "size 2" generally used for standard photography of adults, and an imaging plate called "size 3" generally used for bite wing method photography of adults. In addition, the imaging plate having a large size is specifically an imaging plate having a size larger than "size 3" generally used for occlusal photography of children and adults in dental X-ray photography.

When a plurality of imaging plates 2 are used as in the IP assembly 1 of FIG. 1, for example, occlusal photography for a large size which is required to prepare an imaging plate having a size larger than the size 3 in the conventional case can be performed even with an imaging plate having a small size.

As described above, when the IP assembly 1 including the plurality of imaging plates 2 having a relatively large photographing region is arranged in the oral cavity, the entire IP assembly 1 may not fit in the oral cavity, and a part of the IP assembly 1 may be located outside the oral cavity. That is, a part of the IP holding member 5 may be located outside the oral cavity. In a state where the IP assembly 1 is arranged in the oral cavity, the radiograph formation layer 20 of each imaging plate 2 included in the IP assembly 1 is irradiated with radiation. For example, a tooth radiograph is recorded in the radiograph formation layer 20 of each imaging plate 2 of the IP assembly 1.

The radiograph recorded on the imaging plate 2 is read by a reading apparatus (not illustrated). For example, the imaging plates 2 included in the IP assembly 1 are input to the reading apparatus one by one. The reading apparatus reads a radiograph from the radiograph formation layer 20 of the input imaging plate 2, and generates image data representing the read radiograph. The reading apparatus combines a plurality of pieces of image data indicating the respective radiographs read from the plurality of imaging plates 2 included in the IP assembly 1 to generate composite image data indicating one occlusal image representing the state in the oral cavity. The occlusal image indicated by the composite image data is displayed, for example, on a display unit included in the reading apparatus. Hereinafter, the front surface (in other words, the surface on the front side) of the imaging plate 2 is referred to as a reading target surface 21. The back surface of the imaging plate 2 is referred to as a non-reading surface 22.

The cover member 6 is a member that protects the imaging plate 2. For example, at least a part of the cover member 6 (a region in contact with the reading target surface 21) is made of a radiation transmissive material. The cover member 6 is made of, for example, a material having a light shielding property against visible light. The cover member 6 is made of paper, for example. When hard paper such as cardboard having a constant thickness is used as the material of the cover member 6, mechanical damage of the imaging plate 2 can be reduced. The cover member 6 has, for example, a structure in which a sheet-like member is bent, and is openable and closable. The cover member 6 includes, for example, a sheet-like first cover portion 61 and a sheet-like second cover portion 62 connected to the first cover portion 61. The second cover portion 62 can be opened and closed relatively to the first cover portion 61 with a connection portion with the first cover portion 61 as a base point (in other words, an opening and closing axis).

When no external force is applied to the cover member 6, the second cover portion 62 is slightly opened relatively to the first cover portion 61, and the cover member 6 is opened. Then, when an external force is applied to at least one of the first cover portion 61 and the second cover portion 62 such that the first cover portion 61 and the second cover portion 62 approach each other, the cover member 6 is closed, and the imaging plate 2 is sandwiched between the first cover portion 61 and the second cover portion 62. For example, when the first cover portion 61 and the second cover portion 62 are sandwiched by fingers from the outside thereof, the cover member 6 is closed. When no external force is applied to the cover member 6, the second cover portion 62 may be closed relatively to the first cover portion 61. A user can open it with his/her fingers if necessary.

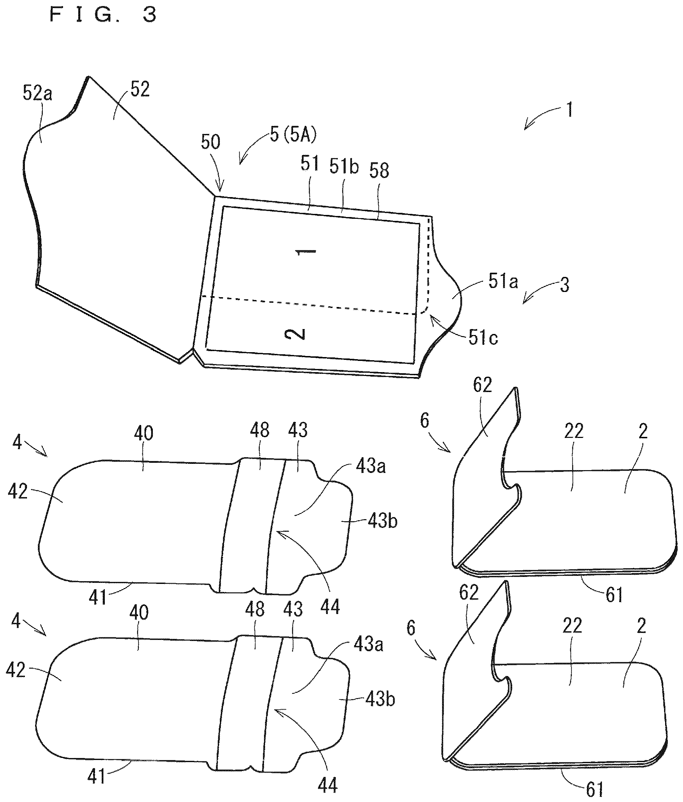

When the imaging plate 2 is sandwiched by the cover member 6, the cover member 6 and the imaging plate 2 are used in combination such that the first cover portion 61 and the second cover portion 62 face the reading target surface 21 and the non-reading surface 22 of the imaging plate 2, respectively. In a state where the imaging plate 2 is sandwiched by the cover member 6, the first cover portion 61 covers the entire region of the reading target surface 21 of the imaging plate 2, and the second cover portion 62 covers a part of the non-reading surface 22 of the imaging plate 2. FIG. 3 is a schematic view illustrating an example of a state in which the imaging plate 2 is arranged on the first cover portion 61 of the cover member 6 in an open state.

Since the imaging plate 2 is covered with the cover member 6 in this manner, the imaging plate 2 is less likely to be mechanically damaged. For example, at the time of dental X-ray photography, the imaging plate 2 is less likely to be damaged when the IP assembly 1 arranged in the oral cavity is bitten by human teeth or when the IP assembly 1 is pressed against and curved along the dentition. The cover member 6 is also called, for example, a bite cover, an IP cover, a carton, or the like.

In addition, since the imaging plate 2 is covered with the cover member 6 made of a material such as paper that hardly transmits visible light, visible light or external light emitted from a surrounding lighting apparatus (for example, a fluorescent lamp or a light-emitting diode (LED)) hardly hits the imaging plate 2. Therefore, the information of the radiograph is hardly lost from the imaging plate 2.

Note that the cover member 6 may be made of a material other than paper. For example, the material of the cover member 6 may be a resin material molded with flexibility, or a composite material of paper and resin having waterproofness or strength in which the surface is covered with resin. In addition, the second cover portion 62 may cover the entire region of the non-reading surface 22.

The waterproof bag 4 is a protective material for the imaging plate 2 and the cover member 6. The waterproof bag 4 is configured so that a fluid such as body fluid (saliva, blood, virus, bacteria, etc.) does not permeate or ooze into the imaging plate 2 and the cover member 6 arranged in the oral cavity of a living organism as a subject (a person to be photographed) at the time of photographing. In addition, the waterproof bag 4 is configured such that an antiseptic solution or the like does not enter the inside of the waterproof bag 4 when the entire waterproof bag 4 is cleaned after photographing. The waterproof bag 4 is basically disposable, and is discarded when used once, for example. The waterproof bag 4 is also called, for example, an (IP) protective bag, a hygienic pouch, or the like. The waterproof bag 4 includes, for example, a bag-shaped main body portion 40 having waterproofness, and a sticky member 48 (also referred to as a pressure-sensitive adhesive 48) for sealing the main body portion 40. The sticky member 48 is provided in the main body portion 40. The sticky member 48 is made of, for example, a radiation transmissive material. The sticky member 48 is made of, for example, a double-sided tape. The sticky member 48 has an adhesive surface to which a part of the main body portion 40 is attached. In the unused waterproof bag 4, for example, the adhesive surface of the sticky member 48 is covered with release paper/liner or a release film (also referred to as a protective sheet), which is a sheet that protects the adhesive surface of the sticky member 48 and can be removed from the adhesive surface to allow adhesion.

The main body portion 40 is, for example, a sanitary member and is made of a biocompatible material. The main body portion 40 is made of, for example, a radiation transmissive material. The main body portion 40 is made of a soft material such as resin, for example. The main body portion 40 may be made of resin such as polyvinyl chloride (PVC) or ethylene-vinylacetate copolymer (EVA), for example. The main body portion 40 has an opening 44 through which the imaging plate 2 is taken in and out.

The main body portion 40 includes, for example, a sheet-like substantially rectangular first cover portion 41 facing one of the reading target surface 21 and the non-reading surface 22 of the imaging plate 2, and a sheet-like substantially rectangular second cover portion 42 facing the other of the reading target surface 21 and the non-reading surface 22. The first cover portion 41 and the second cover portion 42 form a bag shape by connecting their peripheral edges to each other. One end of the first cover portion 41 in the longitudinal direction and one end of the second cover portion 42 in the longitudinal direction are not connected to each other, and constitute the opening 44. The main body portion 40 has, for example, a lid portion 43 capable of closing the opening 44. The lid portion 43 is connected to one end of the first cover portion 41 in the longitudinal direction. The sticky member 48 is provided at an end portion of the second cover portion 42 on the opening 44 side in the longitudinal direction. The lid portion 43 includes an attachment target portion 43a and a pinching portion 43b attached to the adhesive surface of the sticky member 48. An assembly worker of the IP assembly 1 bends the lid portion 43 toward the sticky member 48 while pinching the pinching portion 43b of the lid portion 43 with fingers, and attaches the attachment target portion 43a to the adhesive surface of the sticky member 48.

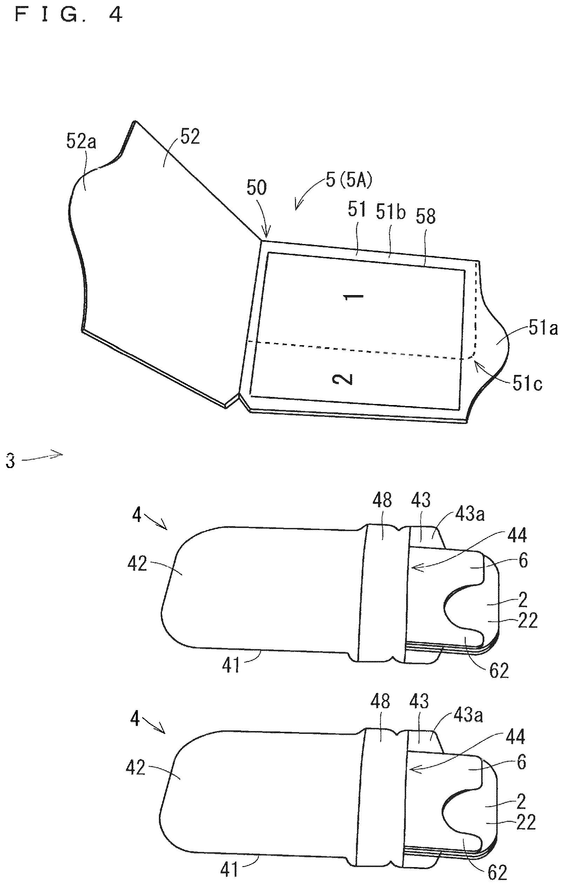

For example, the imaging plate 2 is put in the main body portion 40 from the opening 44 and accommodated in a state of being sandwiched by the cover member 6. For example, the assembly worker holds the cover member 6 in a state where the first cover portion 61 and the second cover portion 62 are sandwiched between two fingers from the outside, and puts the imaging plate 2 and the cover member 6 into the main body portion 40 from the opening 44. FIG. 4 is a schematic view illustrating an example of a state in which the imaging plate 2 and the cover member 6 are put in the main body portion 40 from the opening 44. For example, the cover member 6 is put in the main body portion 40 from the connection side between the first cover portion 61 and the second cover portion 62. The imaging plate 2 covered with the cover member 6 is put in the main body portion 40 from the opening 44 such that the reading target surface 21 and the non-reading surface 22 face the first cover portion 41 and the second cover portion 42 of the main body portion 40, respectively. In a state where the imaging plate 2 and the cover member 6 are accommodated in the main body portion 40, the first cover portion 61 and the second cover portion 62 of the cover member 6 face the first cover portion 41 and the second cover portion 42 of the main body portion 40, respectively. In a state where the imaging plate 2 and the cover member 6 are accommodated in the main body portion 40, the first cover portion 61 and the second cover portion 62 of the cover member 6 may face the second cover portion 42 and the first cover portion 41 of the main body portion 40, respectively. In a state where the imaging plate 2 and the cover member 6 are accommodated in the main body portion 40, a connection portion (opening and closing axis) between the second cover portion 62 and the first cover portion 61 may be located on the opening 44 side.

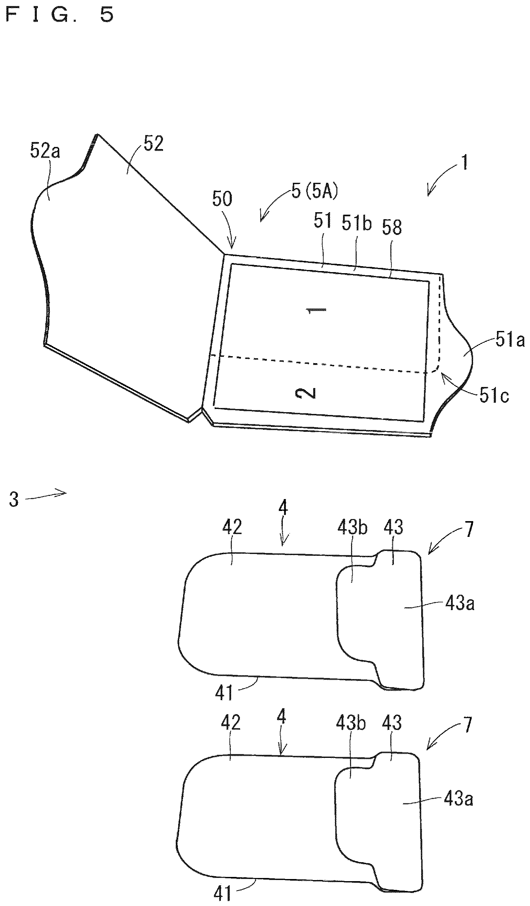

After the imaging plate 2 and the cover member 6 are accommodated in the main body portion 40, the release paper or the release film covering the adhesive surface of the sticky member 48 is removed. Then, the assembly worker bends the lid portion 43 toward the second cover portion 42 while pinching the pinching portion 43b of the lid portion 43 with fingers, and attaches the attachment target portion 43a of the lid portion 43 to the sticky member 48 on the adhesive surface. As a result, the opening 44 is covered with the lid portion 43, and the main body portion 40 is sealed in a watertight manner. As a result, an assembly 7 (also referred to as a unit assembly 7) including the imaging plate 2, the cover member 6 covering the imaging plate 2, and the waterproof bag 4 accommodating the imaging plate 2 and the cover member 6 has been assembled. FIG. 5 is a schematic view illustrating an example of a state in which the opening 44 is covered with the lid portion 43 and the main body portion 40 is sealed. Two unit assemblies 7 are illustrated in FIG. 5. The assembly worker assembles a plurality of unit assemblies 7. When the protection assembly 3 does not include the cover member 6, the unit assembly 7 does not include the cover member 6.

By accommodating the imaging plate 2 in the waterproof bag 4 in this manner, the imaging plate 2 is less likely to be contaminated with saliva, blood, or the like when the IP assembly 1 is arranged in the oral cavity. This makes cross-infection less likely to occur.

Note that the sticky member 48 may be, for example, an application-type adhesive agent instead of the double-sided tape. Instead of the sticky member 48, for example, a curable adhesive may be used. The main body portion 40 may be sealed by bending the lid portion 43 and welding it to the second cover portion 42. Alternatively, the vicinity of the opening 44 of the main body portion 40 may be welded by heat to seal the main body portion 40 in a watertight manner. In addition, the opening 44 may be sealed with a chuck-type structure such as a freezer bag, and the main body portion 40 may be sealed in a watertight manner.

Furthermore, at least a part of the main body portion 40 may have a light shielding property against visible light. In this case, for example, visible light or external light emitted from a surrounding lighting apparatus is less likely to hit the imaging plate 2, so that information of a radiograph is less likely to be lost from the imaging plate 2. As a method of imparting a light shielding property against visible light to at least a part of the main body portion 40, for example, a surface of at least a part of the main body portion 40 may be black. In this case, for example, at least one of the inner surface and the outer surface of the first cover portion 41 of the main body portion 40 may be black. This makes surrounding visible light less likely to hit the imaging plate 2, particularly the radiograph formation layer 20. In addition, at least one of the inner surface and the outer surface of the second cover portion 42 of the main body portion 40 may be black. Even in this case, the surrounding visible light is less likely to hit the imaging plate 2. At least one of both surfaces of the lid portion 43 may be black. In this case, the surrounding visible light is less likely to hit the imaging plate 2. The assembly work of the unit assembly 7 may be performed such that at least a portion having a light shielding property of the waterproof bag 4 and the reading target surface 21 of the imaging plate 2 face each other.

Note that the waterproof bag 4 may include only the bag-shaped main body portion 40 without including the lid portion 43, for example. In this case, the sticky member 48 may be provided, for example, at an end portion on the opening 44 side of the inner surface of the first cover portion 41 of the main body portion 40. Alternatively, the sticky member 48 may be provided, for example, at an end portion on the opening 44 side of the inner surface of the second cover portion 42 of the main body portion 40. In the main body portion 40, in the vicinity of the opening 44, the inner surface of the first cover portion 41 and the inner surface of the second cover portion 42 are stuck to each other by the sticky member 48, and the main body portion 40 is sealed in a watertight manner.

The IP holding member 5 holds the plurality of unit assemblies 7 at predetermined positions. The IP holding member 5 is also called, for example, an (IP) positioning tool, a container, or the like. For example, the IP holding member 5 holds the plurality of unit assemblies 7 such that the plurality of unit assemblies 7 are arranged in a line in a predetermined direction. It can also be said that the IP holding member 5 holds the plurality of imaging plates 2 such that the plurality of imaging plates 2 are arranged in a line in a predetermined direction. In other words, the IP holding member 5 holds the plurality of waterproof bags 4 such that the plurality of waterproof bags 4 are arranged in a line in a predetermined direction.

In addition, the IP holding member 5 holds the plurality of imaging plates 2 such that two adjacent imaging plates 2 partially overlap. For example, the IP holding member 5 holds the plurality of imaging plates 2 such that end portions in the short direction (in other words, end portions on the long side) of two adjacent imaging plates 2 overlap each other.

In the present disclosure, a plurality of types of IP holding members 5 will be described. Hereinafter, an example of the IP holding member 5 illustrated in FIGS. 1 to 9 is referred to as the IP holding member 5A.

The IP holding member 5A is openable and closable. FIG. 1 illustrates the IP holding member 5A in an open state, and FIG. 2 illustrates the IP holding member 5A in a closed state. the IP holding member 5A includes, for example, a base member 50 that covers the plurality of unit assemblies 7 and an attachment portion 58 that attaches the plurality of unit assemblies 7 to the base member 50. The base member 50 covers the plurality of waterproof bags 4. The base member 50 can also be referred to as a main body portion.

The base member 50 has, for example, a structure in which a sheet-like member is bent, and is openable and closable. The base member 50 includes, for example, the fixed portion 51 on which the plurality of unit assemblies 7 are arranged and fixed, and the cover portion 52 that covers the plurality of unit assemblies 7 fixed to the fixed portion 51. The plurality of unit assemblies 7 are fixed to the fixed portion 51 by the attachment portion 58. The waterproof bags 4 of the plurality of unit assemblies 7 are fixed to the fixed portion 51. The cover portion 52 covers the plurality of waterproof bags 4 fixed to the fixed portion 51. The attachment portion 58 attaches the fixed portion 51 and the plurality of waterproof bags 4 to each other.

Each of the fixed portion 51 and the cover portion 52 has, for example, a sheet shape. The cover portion 52 is connected to the fixed portion 51, and is openable and closable relatively to the fixed portion 51 with a connection portion with the fixed portion 51 as a base point (in other words, an opening and closing axis).

When no external force is applied to the base member 50, for example, the cover portion 52 is opened relatively to the fixed portion 51, and the base member 50 is in an open state. Then, when an external force is applied to at least one of the fixed portion 51 and the cover portion 52 such that the fixed portion 51 and the cover portion 52 approach each other, the base member 50 is closed, and the plurality of unit assemblies 7 are sandwiched between the fixed portion 51 and the cover portion 52. For example, when the fixed portion 51 and the cover portion 52 are sandwiched between fingers from the outside, the base member 50 is closed. In a state where the plurality of unit assemblies 7 are sandwiched between the fixed portion 51 and the cover portion 52, the base member 50 faces both surfaces (that is, the reading target surfaces 21 and the non-reading surfaces 22) of the imaging plates 2 of the plurality of unit assemblies 7. The base member 50 may be in a closed state when no external force is applied.

The fixed portion 51 and the cover portion 52 have a pair of pinching portions 51a and 52a, respectively. The pinching portion 51a is provided at an end of the fixed portion 51 opposite to a connection end with the cover portion 52. The pinching portion 52a is provided at an end of the cover portion 52 opposite to a connection end with the fixed portion 51. When the base member 50 is in the closed state, the pair of pinching portions 51a and 52a faces each other. The assembly worker can maintain the closed state of the base member 50 by pinching the pair of pinching portions 51a and 52a with fingers from the outside. When the base member 50 is closed in a state where the unit assembly 7 is fixed to the fixed portion 51, the imaging plate 2 of the unit assembly 7 is not located between the pair of pinching portions 51a and 52a. In the configuration of the IP holding member 5, the pinching portions 51a and 52a may not be provided.

The base member 50 is, for example, a hygienic member, and is made of a biocompatible material (for example, a material conforming to the ISO standard 10993-1 or the like). The base member 50 is made of, for example, a radiation transmissive material. The base member 50 is made of a soft material such as resin, for example. The base member 50 may be made of resin such as PVC or EVA. The base member 50 may be made of the same material as that of the main body portion 40 of the waterproof bag 4, or may be made of a material different from that of the main body portion 40. The base member 50 may be made of paper instead of resin. The base member 50 may be made of paper and resin. In this case, for example, the base member 50 may be formed by coating a base member (also referred to as a main body) made of paper with a resin such as vinyl. As a material of the base member 50, various materials having high radiation permeability such as a carbon fiber material can be used. the IP holding member 5 having the base member 50 may be manufactured in a reusable specification, or the IP holding member 5 may be manufactured in a disposable specification, for example, such that the IP holding member 5 is discarded after being used once.

On the inner surface 51b of the fixed portion 51, that is, the fixed surface 51b to which the plurality of unit assemblies 7 are fixed, arrangement place information 51c indicating arrangement places of the plurality of unit assemblies 7 is indicated by printing or the like. The arrangement place information 51c indicates arrangement places of the plurality of waterproof bags 4 accommodating one-to-one the plurality of imaging plates 2. In the example of FIGS. 1 to 5, the arrangement place information 51c includes numbers 1 and 2 indicating that the two unit assemblies 7 are arranged on the fixed surface 51b. The arrangement direction of the numbers 1 and 2 indicates the arrangement direction of the two unit assemblies 7. The place indicated by the number 1 indicates a schematic arrangement place of one unit assembly 7, and the place indicated by the number 2 indicates a schematic arrangement place of the other unit assembly 7. The arrangement place information 51c also includes a dotted line indicating a detailed arrangement place of one unit assembly 7. Note that the configuration of the arrangement place information 51c is not limited to this configuration. The arrangement place information 51c may indicate the positions of three or more unit assemblies 7, or may indicate the arrangement direction of three or more unit assemblies 7. Furthermore, the arrangement place information 51c may be indicated using various line types, or may be indicated using characters, figures, marks, or the like. The arrangement place information 51c may be any configuration as long as it can indicate, to the user, at least the arrangement of the imaging plate 2 suitable for the photographing method using the IP assembly 1.

The attachment portion 58 for attaching the fixed portion 51 and the plurality of waterproof bags 4 to each other is made of, for example, a radiation transmissive material. Before the plurality of unit assemblies 7 are fixed to the fixed portion 51, that is, in the unused IP holding member 5A, the attachment portion 58 is provided, for example, on the fixed surface 51b. The attachment portion 58 is, for example, a sticky member. As the sticky member, for example, a translucent or transparent double-sided tape is adopted. In the unused IP holding member 5A, release paper/liner or a release film may be stuck to the attachment portion 58. The attachment portion 58 is provided on at least a part of the fixed surface 51b. In the example of FIGS. 1 to 5, the attachment portion 58 is provided on most of the fixed surface 51b. The arrangement place information 51c is seen through the attachment portion 58. Each of the plurality of unit assemblies 7 is, for example, detachable from the fixed surface 51b.

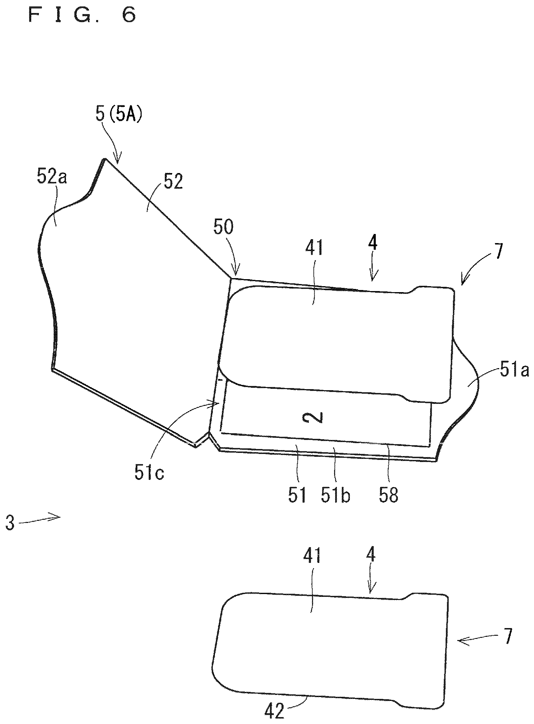

For example, when the two unit assemblies 7 are fixed to the fixed portion 51 of the IP holding member 5A, first, one unit assembly 7 is arranged and fixed in a region indicated by a dotted line included in the arrangement place information 51c in the fixed surface 51b. FIG. 6 is a schematic view illustrating an example of this state. For example, the unit assembly 7 is fixed to the fixed surface 51b such that the non-reading surface 22 of the imaging plate 2 included in the unit assembly 7 is located on the fixed surface 51b side. In addition, the unit assembly 7 is fixed to the fixed surface 51b such that, for example, the bottom of the waterproof bag 4 of the unit assembly 7 is located on the connection end side between the cover portion 52 and the fixed portion 51, and the lid portion 43 of the waterproof bag 4 is located on the pinching portion 51a side.

After one unit assembly 7 is fixed to the fixed surface 51b, the other unit assembly 7 is arranged and fixed to the fixed surface 51b. The assembly of the IP assembly 1 is completed by fixing the plurality of unit assemblies 7 to the fixed surface 51b.

The unit assembly 7 may be fixed to the fixed surface 51b such that the reading target surface 21 of the imaging plate 2 included in the unit assembly 7 is located on the fixed surface 51b side. **In** addition, the unit assembly 7 may be fixed to the fixed surface 51b such that the bottom of the waterproof bag 4 of the unit assembly 7 is located on the pinching portion 51a side and the lid portion 43 of the waterproof bag 4 is located on the connection end side between the cover portion 52 and the fixed portion 51.

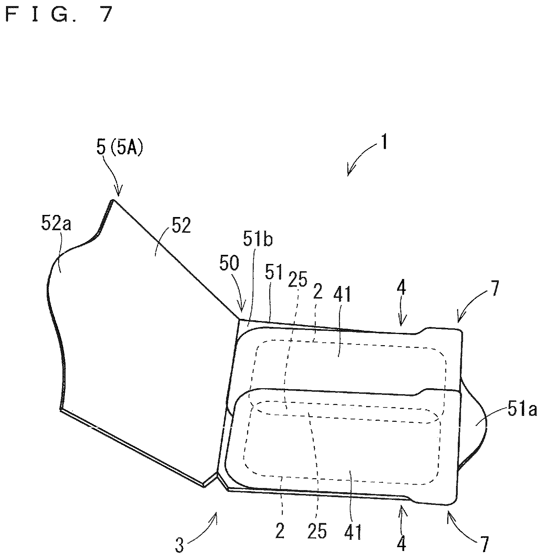

FIG. 7 is a schematic view illustrating an example of the IP assembly 1 assembled. In FIG. 7, the imaging plates 2 are indicated by broken lines. The two unit assemblies 7 are fixed to the fixed portion 51 such that the two imaging plates 2 respectively included in the two unit assemblies 7 partially overlap each other. For example, the two unit assemblies 7 are fixed to the fixed surface 51b by the attachment portion 58 such that one end portion 25 in the short direction of the imaging plate 2 of the unit assembly 7 to be fixed second is located on one end portion 25 in the short direction of the imaging plate 2 of the unit assembly 7 to be fixed first.

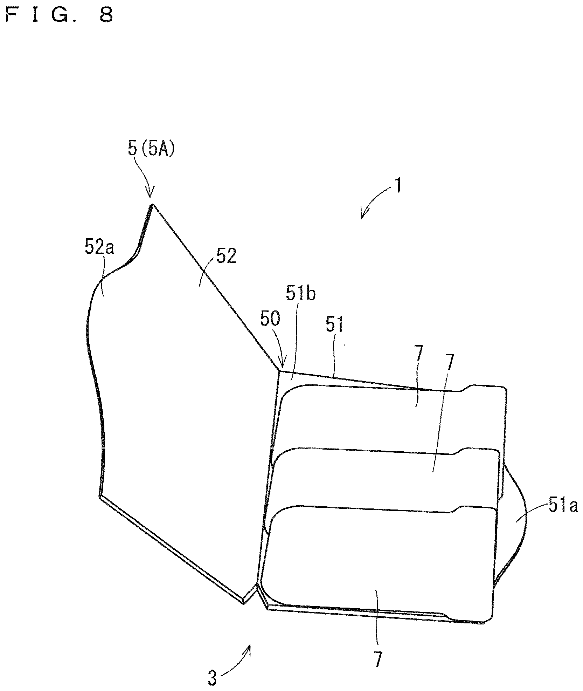

Even when three or more unit assemblies 7 are fixed to the fixed portion 51, each unit assembly 7 is sequentially fixed to the fixed portion 51. Specifically, after the first unit assembly 7 is fixed to the fixed portion 51, the second unit assembly 7 is fixed to the fixed portion 51 such that one end portion in the short direction of the imaging plate 2 of the second unit assembly 7 overlaps one end portion in the short direction of the imaging plate 2 of the first unit assembly 7. Next, the third unit assembly 7 is fixed to the fixed portion 51 such that one end portion in the short direction of the imaging plate 2 of the third unit assembly 7 overlaps the other end portion in the short direction of the imaging plate 2 of the second unit assembly 7. Thereafter, similarly, the fourth and subsequent unit assemblies 7 are sequentially fixed to the fixed portion 51. FIG. 8 is a schematic view illustrating an example of a state in which three unit assemblies 7 are fixed to the fixed portion 51. FIG. 8 illustrates an example of an **IP** assembly 1 including three unit assemblies 7.

The plurality of unit assemblies 7 may be fixed to the fixed portion 51 such that two adjacent imaging plates 2 do not overlap each other. That is, the **IP** holding member 5A may hold the plurality of unit assemblies 7 such that two adjacent imaging plates 2 do not overlap each other.

The configuration of the attachment portion 58 is not limited to the above example. For example, the attachment portion 58 may include a plurality of sticky members (for example, double-sided tapes, application-type adhesive agents, or the like) respectively provided in the plurality of waterproof bags 4 fixed to the fixed portion 51. **In** addition, the attachment portion 58 may include a plurality of sticky members provided on the plurality of waterproof bags 4, respectively, and a sticky member provided on the fixed surface 51b. By providing the sticky member on at least one of the waterproof bag 4 and the fixed portion 51, the waterproof bag 4 can be fixed to the fixed portion 51. That is, the **IP** holding member 5A can hold the waterproof bag 4.

The attachment portion 58 may include a hook-and-loop fastener. **In** this case, the attachment portion 58 may include, for example, a first hook-and-loop fastener provided on the waterproof bag 4 and a second hook-and-loop fastener provided on the fixed surface 51b to be engaged with the first hook-and-loop fastener. When the first hook-and-loop fastener is a male type, the second hook-and-loop fastener is a female type, and when the first hook-and-loop fastener is a female type, the second hook-and-loop fastener is a male type. When the fixed portion 51 and the waterproof bag 4 are attached to each other using the hook-and-loop fasteners, the waterproof bag 4 is detachable from the fixed portion 51. The first hook-and-loop fastener may be provided in all of the plurality of waterproof bags 4, or may be provided in some of the plurality of waterproof bags 4. The waterproof bag 4 not provided with the first hook-and-loop fastener may be fixed to the fixed portion 51 using, for example, a sticky member.

**In** addition, the attachment portion 58 may include a suction board. **In** this case, the attachment portion 58 may include, for example, a suction board that is provided in the waterproof bag 4 and is sucked to the fixed portion 51, or may include a suction board that is provided in the fixed portion 51 and is sucked to the waterproof bag 4. All of the plurality of waterproof bags 4 may be fixed to the fixed portion 51 using a suction board, or some of the plurality of waterproof bags 4 may be fixed to the fixed portion 51 using a suction board. The waterproof bag 4 in which the suction board is not used for fixing to the fixed portion 51 may be fixed to the fixed portion 51 using, for example, a sticky member, or may be fixed to the fixed portion 51 using a hook-and-loop fastener. When the fixed portion 51 and the waterproof bag 4 are attached to each other using the suction board, the waterproof bag 4 is detachable from the fixed portion 51.

At least one of the plurality of waterproof bags 4 fixed to the fixed portion 51 may be undetachable from the fixed portion 51. For example, the attachment portion 58 may include a curable adhesive, and the waterproof bag 4 and the fixed portion 51 may be attached to each other with the adhesive, so that the waterproof bag 4 is not attachable to and detachable from the fixed portion 51. Alternatively, even when the attachment portion 58 includes an adhesive agent having a high adhesive force, the waterproof bag 4 can be designed not to be peeled off from the fixed portion 51 as described above.

If the attachment portion 58 is provided in a part between the base member 50 and the waterproof bag 4, the base member 50 and the waterproof bag 4 can be attached to each other. Therefore, the attachment portion 58 may be provided in at least one of the cover portion 52, the first cover portion 41 of the waterproof bag 4, and the second cover portion 42 of the waterproof bag 4 in FIGS. 1 to 8 according to the method of arranging the unit assemblies 7 with respect to the IP holding member 5.

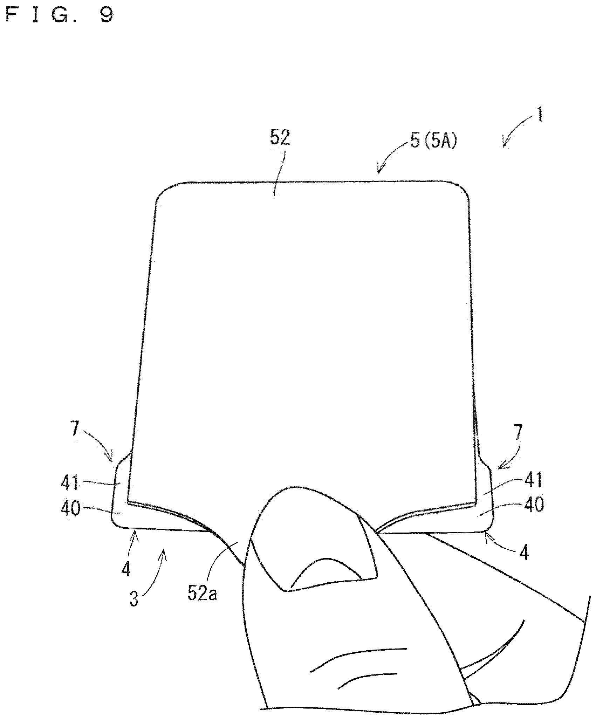

When the assembled IP assembly 1 is arranged in the oral cavity of a person for photographing, as illustrated in FIG. 9, a person in charge of photographing pinches the pair of pinching portions 51a and 52a of the base member 50 of the IP holding member 5A with fingers from the outside. By the pair of pinching portions 51a and 52b being pinched, the unit assembly 7 is hardly pushed by the finger, and the imaging plate 2 is less likely to be damaged. Then, the person in charge of photographing puts the IP assembly 1 into the oral cavity of the subject while pinching the pair of pinching portions 51a and 52a. When the subject bites the base member 50 of the IP holding member 5A in the oral cavity, the closed state of the base member 50 is maintained. At this time, the subject may bite with an unexpected strong force. Since the imaging plate 2 is covered with the cover member 6, the waterproof bag 4, and the IP holding member 5A, the imaging plate 2 is less likely to be damaged even if the base member 50 is bitten with a force greater than or equal to the strength required for photographing. After the IP assembly 1 is placed in the oral cavity, the IP assembly 1 is irradiated with radiation to perform photographing. As a result, the radiograph is recorded in the radiograph formation layer 20 of each imaging plate 2 of the IP assembly 1.

When the photographing is completed, the IP assembly 1 is taken out from the oral cavity of the subject. Then, the plurality of unit assemblies 7 are removed from the fixed portion 51 of the IP holding member 5A. the IP holding member 5A from which the plurality of unit assemblies 7 have been removed may be discarded or reused. That is, the IP holding member 5A may be disposable or may be reused. Next, the imaging plate 2 covered with the cover member 6 is taken out from the waterproof bag 4 of each unit assembly 7. At this time, for example, the operator pinches the pinching portion 43b of the waterproof bag 4 and turns the lid portion 43 to expose the opening 44, and then takes out the imaging plate 2 covered with the cover member 6 from the opening 44. When the waterproof bag 4 is not detachable from the fixed portion 51, the imaging plate 2 covered with the cover member 6 may be taken out from the waterproof bag 4 of the unit assembly 7 fixed to the fixed portion 51.

The imaging plate 2 taken out from the waterproof bag 4 is carried to an insertion port of the reading apparatus while being sandwiched by the cover member 6. Thereafter, the cover member 6 is opened, and the imaging plate 2 is inserted into the insertion port. The reading apparatus reads a radiograph from the inserted imaging plate 2. Similarly, a radiograph is read from each imaging plate 2 included in the IP assembly 1. The reading apparatus transmits a plurality of pieces of image data indicating the respective radiographs read from the plurality of imaging plates 2 included in the IP assembly 1 to, for example, an external computer apparatus. The computer apparatus combines a plurality of pieces of image data from the reading apparatus to generate combined image data indicating one occlusal image. Note that a computer apparatus included in the reading apparatus may combine a plurality of pieces of image data to generate composite image data indicating one occlusal image.

When one imaging plate 2 is used for photographing due to a narrow photographing range or the like, the unit assembly 7 is arranged alone in the oral cavity, and the unit assembly 7 is irradiated with radiation.

As described above, the imaging plate protection assembly 3 includes the plurality of waterproof bags 4 accommodating one-to-one the plurality of imaging plates 2. As a result, unlike the technique of Japanese Patent Application Laid-Open No. 2011-212401 described above, the waterproof bags 4 having the same size can be used regardless of the number of the plurality of imaging plates 2 included in the medical imaging assembly 1. Therefore, the number of types of sizes of the waterproof bag 4 can be reduced. As a result, the cost of the imaging plate protection assembly 3 can be reduced. In addition, the waterproof bag 4 can be easily managed.

In addition, since the waterproof bag 4 having the same size as the waterproof bag 4 used when one imaging plate 2 is used for photographing can also be used in the imaging plate protection assembly 3, the number of types of sizes of the waterproof bag 4 can be reduced also from this point.

In the imaging plate protection assembly 3, since the IP holding member 5 holds the plurality of imaging plates 2 accommodated in the plurality of waterproof bags 4 at predetermined positions, the thickness of the contents in the waterproof bag 4 can be reduced as compared with the case where the entire IP holding member 5 is accommodated in one waterproof bag 4. As a result, the waterproof bag 4 is easily closed, and a gap is less likely to be generated when the waterproof bag 4 is closed, so that body fluid such as saliva is less likely to enter the waterproof bag 4.

When the thickness of the IP holding member is increased, the stability of the positional relationship between the plurality of imaging plates 2 held by the IP holding member can be improved. On the other hand, when one waterproof bag accommodates an IP holding member having a large thickness, the waterproof bag is more difficult to close. Therefore, in a case where one waterproof bag accommodates the IP holding member, when the thickness of the IP holding member is increased, the body fluid more easily enters the waterproof bag.

In the present example, since the IP holding member 5 holds the plurality of imaging plates 2 accommodated in the plurality of waterproof bags 4 at predetermined positions, even if the thickness of the IP holding member 5 is increased in order to improve the stability of the positional relationship between the plurality of imaging plates 2, the waterproof bags 4 can maintain a state in which body fluid such as saliva is less likely to enter the waterproof bag 4 without being affected by the thickness change of the IP holding member 5. Therefore, it is possible to make it difficult for the body fluid to enter the waterproof bag 4 while using the IP holding member 5 capable of holding the plurality of imaging plates 2 in a stable positional relationship.

Further, when the base member 50 of the IP holding member 5A is made of resin, it is possible to make the subject less likely to feel uncomfortable when the IP holding member 5 is put in the oral cavity. When the base member 50 is made of paper, the material cost of the IP holding member 5A can be reduced. Note that the IP holding member 5A may not include the cover portion 52. Even in this case, since each of the fixed portion 51 and the waterproof bag 4 is made of resin, it is possible to make the subject less likely to feel uncomfortable when the IP assembly 1 is put in the oral cavity.

In addition, since the IP holding member 5A is openable and closable, the plurality of waterproof bags 4 can be easily attached to the IP holding member 5A.

In addition, since the IP holding member 5A faces both surfaces of the imaging plate 2, the imaging plate 2 is less likely to be damaged even if the IP assembly 1 is bitten by the subject.

In addition, since the plurality of waterproof bags 4 are attached to the fixed portion 51 of the IP holding member 5A by the attachment portion 58, the plurality of imaging plates 2 can be easily held at predetermined positions.

The configuration of the IP holding member 5 is not limited to the above example. The IP holding member 5 may be, for example, an openable and closable case type. For example, the IP holding member 5 may include a box-shaped resin main body portion having an open upper surface, and a resin lid portion connected to the main body portion and capable of covering the opening of the main body portion, and the lid portion may be openably fitted to the main body portion. In this case, a plurality of pressing protrusions that abut on each unit assembly 7 on the main body portion when the lid portion is fitted to the main body portion may be provided on the inner surface of the lid portion. As a result, when the lid portion is closed and fitted to the main body portion, the plurality of pressing protrusions abut on each unit assembly 7, and the position of each unit assembly 7 in the IP holding member 5 is fixed. As a result, the IP holding member 5 can hold the plurality of unit assemblies 7 at predetermined positions. The arrangement place information indicating the arrangement places of the plurality of unit assemblies 7 may be indicated on the inner surface of the main body portion.

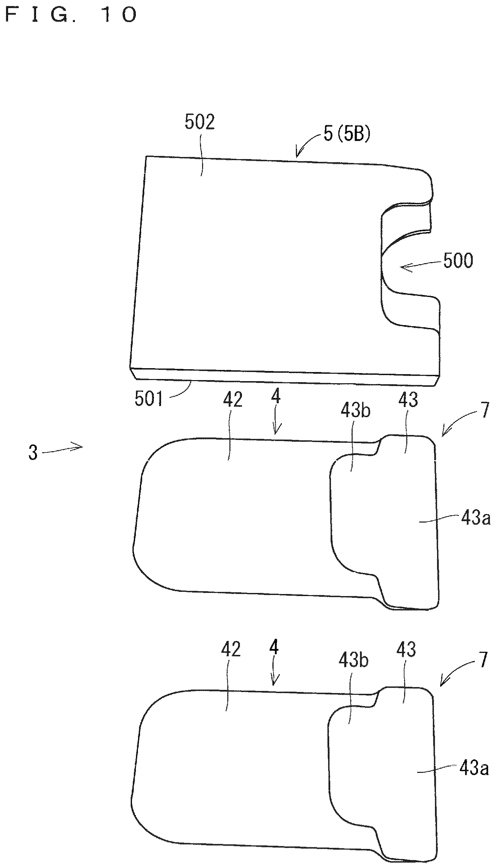

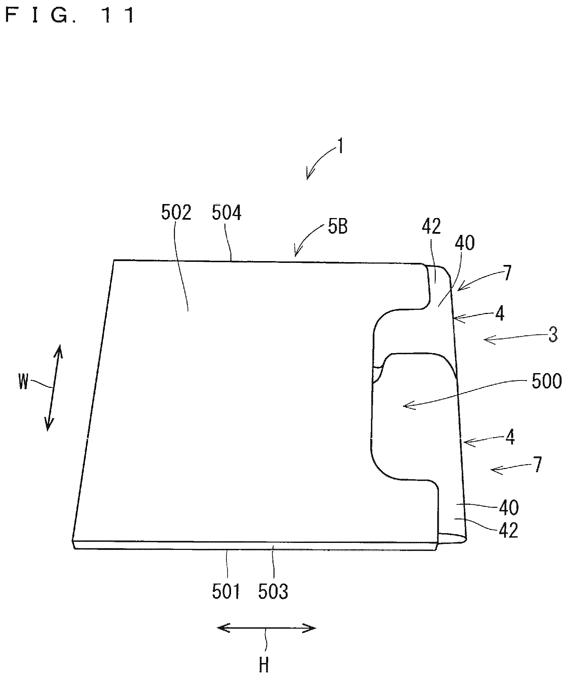

FIGS. 10 and 11 are schematic views illustrating another configuration example of the IP holding member 5. The IP holding member 5 (also referred to as the IP holding member 5B) illustrated in FIGS. 10 and 11 can hold the plurality of unit assemblies 7 at predetermined positions by sandwiching the plurality of unit assemblies 7. The IP holding member 5B can hold, for example, two unit assemblies 7 at predetermined positions. FIG. 10 illustrates the IP holding member 5B before holding the unit assemblies 7. In FIG. 11, the IP holding member 5B holding the two unit assemblies 7 is illustrated. FIG. 11 illustrates the IP assembly 1 after completion of assembly.

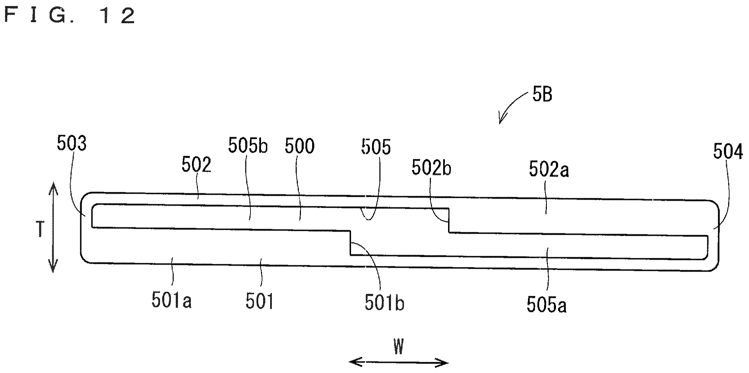

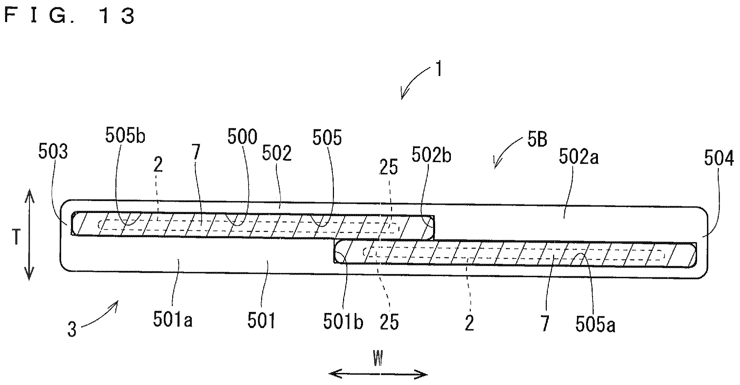

The IP holding member 5B is made of a radiation transmissive material. The IP holding member 5B is made of paper, for example. As illustrated in FIGS. 10 and 11, the IP holding member 5B has, for example, a substantially box shape having a very small thickness. The IP holding member 5B has an opening 500 on an end surface (also referred to as a side surface) thereof through which the unit assembly 7 is taken in and out. The opening 500 is connected to an internal space 505 (see FIGS. 12 and 13 described later) of the IP holding member 5B.

FIGS. 12 and 13 are schematic views illustrating an example of a state in which the IP holding member 5B is viewed from the opening 500 side. FIG. 12 illustrates only the IP holding member 5B, and FIG. 13 illustrates the IP holding member 5B sandwiching the plurality of unit assemblies 7. In FIG. 13, for convenience of description, oblique hatching is illustrated in the unit assemblies 7. In FIG. 13, the imaging plates 2 are indicated by broken lines.

The IP holding member 5B has the main wall portions 501 and 502 facing each other. The main wall portions 501 and 502 function as a pair of a first sandwiching portion 501 and a second sandwiching portion 502 capable of sandwiching the unit assemblies 7, respectively. For example, each unit assembly 7 is inserted into the internal space 505 of the IP holding member 5B from the opening 500 such that the first cover portion 41 and the second cover portion 42 of the waterproof bag 4 face the first sandwiching portion 501 and the second sandwiching portion 502 of the IP holding member 5B, respectively. Each unit assembly 7 is inserted into the IP holding member 5B from the bottom side of the waterproof bag 4. The plurality of unit assemblies 7 inserted into the IP holding member 5B are sandwiched by the first sandwiching portion 501 and the second sandwiching portion 502. Most of the unit assembly 7 is accommodated in the internal space 505 of the IP holding member 5B. Note that the entire unit assembly 7 may be accommodated in the internal space 505.

Hereinafter, with respect to the direction of the IP holding member 5B, a direction along the short direction of the imaging plate 2 of the unit assembly 7 held by the IP holding member 5B as illustrated in FIGS. 11 and 13 is referred to as a width direction W of the IP holding member 5B. A direction perpendicular to the width direction W and the thickness direction T of the IP holding member 5B is referred to as a height direction H of the IP holding member 5B. As illustrated in FIGS. 12 and 13, the left side and the right side (that is, the left side and the right side of FIGS. 12 and 13) when the IP holding member 5B in a posture in which the second sandwiching portion 502 is located on the upper side and the first sandwiching portion 501 is located on the lower side is viewed from the opening 500 side are defined as the left side and the right side of the IP holding member 5B, respectively.

As illustrated in FIGS. 12 and 13, the first sandwiching portion 501 has a large thickness portion 501a whose thickness increases toward the internal space 505. A substantially left half of the first sandwiching portion 501 is the large thickness portion 501a. Specifically, the large thickness portion 501a exists is, in the width direction W, from the left end of the first sandwiching portion 501 to a position slightly on the left side of the center of the first sandwiching portion 501 in the width direction W, and exists over the entire range in the height direction H.

Similarly, the second sandwiching portion 502 has a large thickness portion 502a whose thickness increases toward the internal space 505. A substantially right half of the second sandwiching portion 502 is the large thickness portion 502a. Specifically, the large thickness portion 502a exists, in the width direction W, from the right side end of the second sandwiching portion 502 to a position slightly on the right side of the center of the second sandwiching portion 502 in the width direction W, and exists over the entire range in the height direction H. In the thickness direction T, the large thickness portion 502a does not face the large thickness portion 501a. In the width direction W, a right end surface 501b of the large thickness portion 501a and a left end surface 502b of the large thickness portion 502a are separated from each other.

One unit assembly 7 of the two unit assemblies 7 held by the IP holding member 5B is inserted into a partial space 505a between the right end surface 501b of the large thickness portion 501a of the first sandwiching portion 501 and the inner surface of the right wall portion 504 of the IP holding member 5B in the internal space 505. The one unit assembly 7 is sandwiched between the large thickness portion 502a of the second sandwiching portion 502 and a portion of the first sandwiching portion 501 facing the large thickness portion 502a in the thickness direction T.

The other unit assembly 7 is inserted into a partial space 505b between the left end surface 502b of the large thickness portion 502a of the second sandwiching portion 502 and the inner surface of the left wall portion 503 of the IP holding member 5B in the internal space 505. The other unit assembly 7 is sandwiched between the large thickness portion 501a of the first sandwiching portion 501 and a portion of the second sandwiching portion 502 facing the large thickness portion 501a in the thickness direction T. The partial spaces 505a and 505b are not partitioned, and the partial space 505b and the partial space 505b are connected to each other. As illustrated in FIG. 13, the two unit assemblies 7 are held by the IP holding member 5B such that one end portion 25 in the short direction of the imaging plate 2 of one unit assembly 7 and one end portion 25 in the short direction of the imaging plate 2 of the other unit assembly 7 overlap each other in the thickness direction T, similarly to the case of being held by the IP holding member 5A described above.

As described above, since the IP holding member 5B sandwiches and holds the unit assembly 7, a member for fixing the unit assembly 7 like the attachment portion 58 described above is unnecessary. In addition, since the IP holding member 5B is made of paper, the material cost of the IP holding member 5B can be reduced. Note that the IP holding member 5B may be made of a material other than paper. For example, the IP holding member 5B may be made of resin such as PVC or EVA. In this case, when the subject puts the IP holding member 5B into the oral cavity, the subject is less likely to feel uncomfortable. The IP holding member 5B may be made of paper and resin. In this case, for example, the IP holding member 5B may be formed by coating a base member (also referred to as a main body) made of paper with a resin such as vinyl.

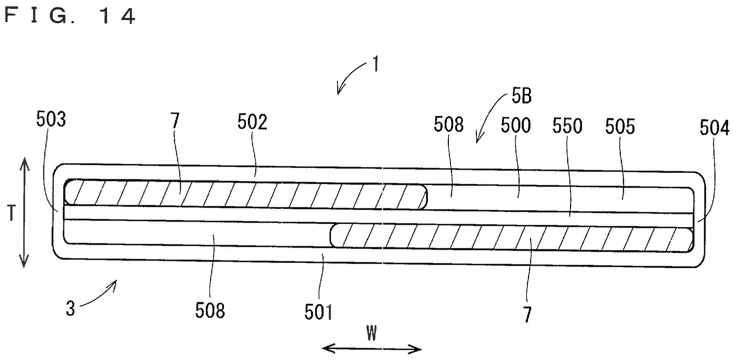

The internal space 505 of the IP holding member 5B may have a plurality of separated spaces 508 partitioned from each other and in which a plurality of unit assemblies 7 are arranged. FIG. 14 is a schematic view illustrating an example of the IP holding member 5B in this case. In the example of FIG. 14, the internal space 505 is partitioned into two separated spaces 508 in the thickness direction T by a partition portion 550. The partition portion 550 extends from the left wall portion 503 to the right wall portion 504. One unit assembly 7 is sandwiched between the main wall portion 501 and the partition portion 550, and the other unit assembly 7 is sandwiched between the main wall portion 502 and the partition portion 550. As a result, the positional relationship between the two unit assemblies 7 is maintained.

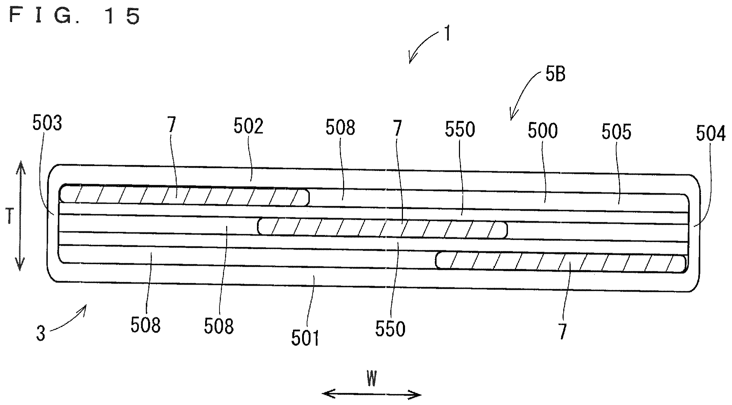

When the IP holding member 5B holds three unit assemblies 7, for example, the internal space 505 may be partitioned into three separated spaces 508 in the thickness direction T by two partition portions 550 as illustrated in FIG. 15. In the case of the example of FIG. 15, one unit assembly 7 is sandwiched between the main wall portion 501 and the partition portion 550, another unit assembly 7 is sandwiched between the two partition portions 550, and still another unit assembly 7 is sandwiched between the main wall portion 502 and the partition portion 550. As another example, one unit assembly 7 may be arranged at each of the left end portion and the right end portion of the partial space 508 at the uppermost stage in FIG. 15, and one unit assembly 7 may be arranged at the central portion of the partial space 508 at the middle stage.

As described above, when the internal space 505 has the plurality of separated spaces 508 in which the plurality of unit assemblies 7 are arranged and partitioned from each other, each unit assembly 7 can be easily put in the internal space 505. As a result, assembling workability of the IP assembly 1 is improved.

In the examples of FIGS. 14 and 15, the width (that is, the dimension in the width direction W) of the partial space 508 is considerably larger than the width of the unit assembly 7 (that is, the width of the waterproof bag 4), but may be set to be substantially the same as the width of the unit assembly 7. For example, in the example of FIG. 14, the width of the partial space 508 may substantially coincide with the width of the waterproof bag 4 of the unit assembly 7 by filling the space of the substantially right half of the upper partial space 508. This facilitates positioning of the unit assembly 7 in the partial space 508. Alternatively, a partition portion that partitions the partial space 508 into two spaces in the width direction W may be provided slightly on the right side of the center of the upper partial space 508 in the width direction W, and the unit assembly 7 may be arranged in the left space of the two spaces.

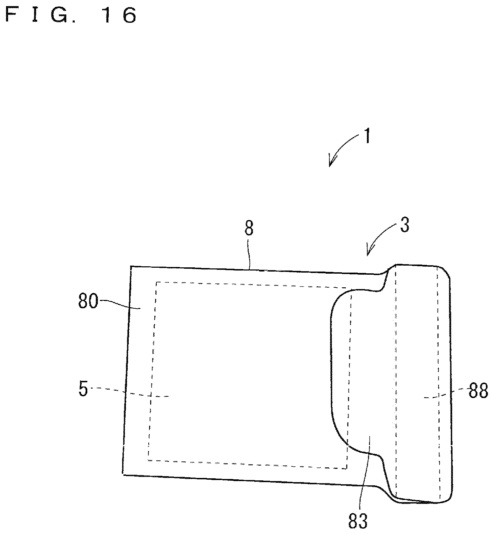

The imaging plate protection assembly 3 may further include a covering member 8 covering the entire IP holding member 5. FIG. 16 is a schematic view illustrating an example of a state in which the entire IP holding member 5 holding the plurality of unit assemblies 7 is covered with the covering member 8.

The covering member 8 has, for example, the same configuration as the waterproof bag 4. The IP holding member 5 is put in a main body portion 80 from an opening of the main body portion 80 of the covering member 8. Then, after the IP holding member 5 is put in the main body portion 80, a lid portion 83 is attached to a sticky member 88 (also referred to as a pressure-sensitive adhesive 88) provided in the main body portion 80. Thus, the main body portion 80 is sealed.

The covering member 8 is made of, for example, a radiation transmissive material. That is, the main body portion 80 and the sticky member 88 are made of, for example, a radiation transmissive material. The sticky member 88 may be a double-sided tape or an application-type adhesive agent. The main body portion 80 may be made of resin such as PVC or EVA, for example. The material of the main body portion 80 may be the same as or different from the material of the main body portion 40 of the waterproof bag 4. When the main body portion 80 is made of resin, it is possible to make the subject less likely to feel uncomfortable when the subject puts the IP assembly 1 into the oral cavity. In addition, since the covering member 8 covers the entire IP holding member 5, body fluid such as saliva is less likely to adhere to the IP holding member 5. This makes it possible to improve ease of handling when the IP holding member 5 and the like inside the covering member 8 are reused.

Instead of the sticky member 88, for example, a curable adhesive may be used. The main body portion 80 may be sealed by welding the bent lid portion 83. Similarly to the main body portion 40 of the waterproof bag 4, at least a part of the main body portion 80 may have a light shielding property against visible light. In this case, for example, visible light or external light emitted from a surrounding lighting apparatus is less likely to hit the imaging plate 2, so that information of a radiograph is less likely to be lost from the imaging plate 2. In addition, the covering member 8 may have a configuration different from that of the waterproof bag 4.

As described above, although the medical imaging assembly and the imaging plate protection assembly have been described in detail, the above description is illustrative in all aspects, and the present disclosure is not limited thereto. In addition, the various examples described above can be applied in combination as long as they do not contradict each other. Then, it is understood that numerous examples not described may be envisioned without departing from the claimed subject-matter.

The present specification and the drawings disclose the following aspects.

An imaging plate protection assembly according to a first aspect is an imaging plate protection assembly capable of accommodating a plurality of imaging plates, the imaging plate protection assembly including: a plurality of waterproof bags accommodating one-to-one the plurality of imaging plates; and an IP holding member that holds the plurality of imaging plates accommodated in the plurality of waterproof bags at predetermined positions.

According to the first aspect, because the imaging plate protection assembly includes the plurality of waterproof bags accommodating one-to-one the plurality of imaging plates, the waterproof bag having the same size can be used regardless of the number of the plurality of imaging plates held by the IP holding member. Accordingly, the number of types of sizes of the waterproof back can be reduced. Furthermore, the thickness of the contents in the waterproof bag can be reduced as compared with the case where the waterproof bag accommodates the entire IP holding member holding the plurality of imaging plates at predetermined positions. As a result, the waterproof bag is easily closed, and body fluid such as saliva is less likely to enter the waterproof bag.

A second aspect is the imaging plate protection assembly according to the first aspect, in which the IP holding member includes: a fixed portion to which the plurality of waterproof bags are fixed; and an attachment portion that attaches the fixed portion and the plurality of waterproof bags to each other. In this case, by attaching the plurality of waterproof bags to the fixed portion by the attachment portion, the plurality of imaging plates are easily held at predetermined positions.

A third aspect is the imaging plate protection assembly according to the second aspect, in which at least one of the plurality of waterproof bags is detachable from the fixed portion. In this case, the waterproof bag can be easily removed.

A fourth aspect is the imaging plate protection assembly according to any one of the first to third aspects, in which the IP holding member faces both surfaces of each of the plurality of imaging plates accommodated in the plurality of waterproof bags. In this case, the imaging plate is less likely to be damaged.

A fifth aspect is the imaging plate protection assembly according to the fourth aspect, in which the IP holding member is openable and closable. In this case, the imaging plate is easily attached to the IP holding member.

A sixth aspect is the imaging plate protection assembly according to the first, fourth, or fifth aspect, in which the IP holding member sandwiches the plurality of imaging plates accommodated in the plurality of waterproof bags. In this case, a member for fixing the imaging plate becomes unnecessary.

A seventh aspect is the imaging plate protection assembly according to the sixth aspect, in which an inner space of the IP holding member has a plurality of separated spaces partitioned from each other, in which the plurality of waterproof bags in which the plurality of imaging plates are accommodated are respectively arranged. In this case, each imaging plate is easily put in the internal space.

An eighth aspect is the imaging plate protection assembly according to any one of the first to seventh aspects, further including a covering member covering the entire IP holding member. In this case, it is possible to make the subject less likely to feel uncomfortable when the imaging plate protection assembly is put in the oral cavity. In this case, body fluid such as saliva is less likely to adhere to the IP holding member. This makes it possible to improve ease of handling when the IP holding member and the like inside the covering member are reused.

A ninth aspect is the imaging plate protection assembly according to any one of the first to eighth aspects, in which the IP holding member is a radiation transmissive material and is made of paper or resin. In this case, when the IP holding member is made of paper, the material cost of the IP holding member can be reduced. When the IP holding member is made of resin, the subject is less likely to feel uncomfortable when the IP holding member is put in the oral cavity.

A medical imaging assembly according to a tenth aspect includes: the imaging plate protection assembly according to any one of the first to ninth aspects; and a plurality of imaging plates accommodated in a plurality of the imaging plate protection assemblies.

While the invention has been shown and described in detail, the foregoing description is in all aspects illustrative and not restrictive.

## Claims

1. An imaging plate protection assembly (3) capable of accommodating a plurality of imaging plates, IP, (2) that are recording media for recording radiographs, the imaging plate protection assembly (3) comprising:
a plurality of waterproof bags (4) configured to accommodate one-to-one the plurality of imaging plates (2) so that it is difficult for fluid to enter inside of the plurality of waterproof bags (4); and
an IP holding member (5, 5A, 5B) configured to hold the plurality of imaging plates (2) accommodated in the plurality of waterproof bags (4) at predetermined positions,
wherein the IP holding member (5, 5A) includes:
a fixed portion (51) to which the plurality of waterproof bags (4) is fixed; and
an attachment portion (58) that attaches the fixed portion (51) and the plurality of waterproof bags (4) to each other.

2. The imaging plate protection assembly (3) according to claim 1, wherein at least one of the plurality of waterproof bags (4) is detachable from the fixed portion (51).

3. The imaging plate protection assembly (3) according to any one of claims 1 to 2, wherein the IP holding member (5, 5A, 5B) faces both surfaces (21, 22) of each of the plurality of imaging plates (2) when they are accommodated in the plurality of waterproof bags (4).

4. The imaging plate protection assembly (3) according to any one of claims 1 to 3, wherein the IP holding member (5, 5A) is openable and closable.

5. An imaging plate protection assembly (3) capable of accommodating a plurality of imaging plates, IP, (2) that are recording media for recording radiographs, the imaging plate protection assembly (3) comprising:
a plurality of waterproof bags (4) configured to accommodate one-to-one the plurality of imaging plates (2) so that it is difficult for fluid to enter inside of the plurality of waterproof bags (4); and
an IP holding member (5, 5A, 5B) configured to hold the plurality of imaging plates (2) accommodated in the plurality of waterproof bags (4) at predetermined positions,
wherein the IP holding member (5, 5B) sandwiches the plurality of imaging plates (2) when they are accommodated in the plurality of waterproof bags (4),
wherein an inner space (505) of the IP holding member (5, 5B) has a plurality of separated spaces (508) partitioned from each other, in which the plurality of waterproof bags (4) in which the plurality of imaging plates (2) can be accommodated are respectively arranged.

6. The imaging plate protection assembly (3) according to any one of claims 1 to 5, further comprising a covering member (8) covering the entire IP holding member (5, 5A, 5B).

7. The imaging plate protection assembly (3) according to any one of claims 1 to 6, wherein the IP holding member (5, 5A, 5B) is a radiation transmissive material and is made of paper or resin.

8. A medical imaging assembly (1) comprising:
the imaging plate protection assembly (3) according to any one of claims 1 to 7; and
a plurality of imaging plates (2) accommodated in the imaging plate protection assembly (3).

## Patentansprüche

1. Bildplatten-Schutzanordnung (3), die in der Lage ist, eine Vielzahl von Bildplatten, IP, (2) aufzunehmen, die Aufzeichnungsmedien zum Aufzeichnen von Röntgenaufnahmen sind, wobei die Bildplatten-Schutzanordnung (3) aufweist:
eine Vielzahl von wasserdichten Beuteln (4), die konfiguriert sind, um die Vielzahl von Bildplatten (2) eins zu eins aufzunehmen, so dass es für Fluid schwierig ist, in das Innere der Vielzahl von wasserdichten Beuteln (4) einzudringen; und
ein IP-Halteelement (5, 5A, 5B), das konfiguriert ist, um die Vielzahl von Bildplatten (2), die in der Vielzahl von wasserdichten Beuteln (4) aufgenommen sind, an vorbestimmten Positionen zu halten,
wobei das IP-Halteelement (5, 5A) umfasst:
einen fixierten Abschnitt (51), an dem die Vielzahl von wasserdichten Beuteln (4) fixiert ist; und
einen Befestigungsabschnitt (58), der den fixierten Abschnitt (51) und die Vielzahl von wasserdichten Beuteln (4) aneinander befestigt.

2. Bildplatten-Schutzanordnung (3) nach Anspruch 1, wobei mindestens einer der Vielzahl von wasserdichten Beuteln (4) von dem fixierten Abschnitt (51) abnehmbar ist.

3. Bildplatten-Schutzanordnung (3) nach einem der Ansprüche 1 bis 2, wobei das IP-Halteelement (5, 5A, 5B) beiden Oberflächen (21, 22) jeder der Vielzahl von Bildplatten (2) zugewandt ist, wenn sie in der Vielzahl von wasserdichten Beuteln (4) aufgenommen sind.

4. Bildplatten-Schutzanordnung (3) nach einem der Ansprüche 1 bis 3, wobei das IP-Halteelement (5, 5A) geöffnet und geschlossen werden kann.

5. Bildplatten-Schutzanordnung (3), die in der Lage ist, eine Vielzahl von Bildplatten, IP, (2) aufzunehmen, die Aufzeichnungsmedien zum Aufzeichnen von Röntgenaufnahmen sind, wobei die Bildplatten-Schutzanordnung (3) aufweist:
eine Vielzahl von wasserdichten Beuteln (4), die konfiguriert sind, um die Vielzahl von Bildplatten (2) eins zu eins aufzunehmen, so dass es für Fluid schwierig ist, in das Innere der Vielzahl von wasserdichten Beuteln (4) einzudringen; und
ein IP-Halteelement (5, 5A, 5B), das konfiguriert ist, um die Vielzahl von Bildplatten (2), die in der Vielzahl von wasserdichten Beuteln (4) aufgenommen sind, an vorbestimmten Positionen zu halten,
wobei das IP-Halteelement (5, 5B) die Vielzahl von Bildplatten (2) sandwichartig aufnimmt, wenn sie in der Vielzahl von wasserdichten Beuteln (4) aufgenommen sind,
wobei ein Innenraum (505) des IP-Halteelements (5, 5B) eine Vielzahl von getrennten Räumen (508) aufweist, die voneinander getrennt sind, in denen die Vielzahl von wasserdichten Beuteln (4), in denen die Vielzahl von Bildplatten (2) aufgenommen werden können, jeweils angeordnet sind.

6. Bildplatten-Schutzanordnung (3) nach einem der Ansprüche 1 bis 5, ferner aufweisend ein Abdeckelement (8), das das gesamte IP-Halteelement (5, 5A, 5B) abdeckt.

7. Bildplatten-Schutzanordnung (3) nach einem der Ansprüche 1 bis 6, wobei das IP-Halteelement (5, 5A, 5B) ein strahlungsdurchlässiges Material ist und aus Papier oder Harz hergestellt ist.

8. Medizinische Bildgebungsanordnung (1), aufweisend:
die Bildplatten-Schutzanordnung (3) nach einem der Ansprüche 1 bis 7; und
eine Vielzahl von Bildplatten (2), die in der Bildplatten-Schutzanordnung (3) aufgenommen sind.

## Revendications

1. Ensemble de protection de plaque d'imagerie (3) apte à loger une pluralité de plaques d'imagerie, IP, (2) qui sont des supports d'enregistrement pour enregistrer des radiographies, l'ensemble de protection de plaque d'imagerie (3) comprenant :
une pluralité de sacs étanches (4) configurés pour loger un à un la pluralité de plaques d'imagerie (2) de telle sorte qu'il soit difficile pour le fluide d'entrer à l'intérieur de la pluralité de sacs étanches (4) ; et
un organe de maintien d'IP (5, 5A, 5B) configuré pour maintenir la pluralité de plaques d'imagerie (2) logées dans la pluralité de sacs étanches (4) en des positions prédéterminées,
dans lequel l'organe de maintien d'IP (5, 5A) comporte :
une partie fixe (51) à laquelle la pluralité de sacs étanches (4) est fixée ; et
une partie d'attache (58) qui attache la partie fixe (51) et la pluralité de sacs étanches (4) l'une aux autres.

2. Ensemble de protection de plaque d'imagerie (3) selon la revendication 1, dans lequel l'un au moins de la pluralité de sacs étanches (4) est détachable de la partie fixe (51).

3. Ensemble de protection de plaque d'imagerie (3) selon l'une des revendications 1 et 2, dans lequel l'organe de maintien d'IP (5, 5A, 5B) fait face aux deux surfaces (21, 22) de chacune de la pluralité de plaques d'imagerie (2) lorsqu'elles sont logées dans la pluralité de sacs étanches (4).

4. Ensemble de protection de plaque d'imagerie (3) selon l'une des revendications 1 à 3, dans lequel l'organe de maintien d'IP (5, 5A) peut être ouvert et fermé.

5. Ensemble de protection de plaque d'imagerie (3) apte à loger une pluralité de plaques d'imagerie, IP, (2) qui sont des supports d'enregistrement pour enregistrer des radiographies, l'ensemble de protection de plaque d'imagerie (3) comprenant :
une pluralité de sacs étanches (4) configurés pour loger un à un la pluralité de plaques d'imagerie (2) de telle sorte qu'il soit difficile pour le fluide d'entrer à l'intérieur de la pluralité de sacs étanches (4) ; et
un organe de maintien d'IP (5, 5A, 5B) configuré pour maintenir la pluralité de plaques d'imagerie (2) logées dans la pluralité de sacs étanches (4) en des positions prédéterminées,
dans lequel l'organe de maintien d'IP (5, 5B) prend en sandwich la pluralité de plaques d'imagerie (2) lorsqu'elles sont logées dans la pluralité de sacs étanches (4),
dans lequel un espace intérieur (505) de l'organe de maintien d'IP (5, 5B) présente une pluralité d'espaces séparés (508) cloisonnés les uns des autres, dans lesquels la pluralité de sacs étanches (4) dans lesquels la pluralité de plaques d'imagerie (2) peuvent être logées sont respectivement agencés.

6. Ensemble de protection de plaque d'imagerie (3) selon l'une des revendications 1 à 5, comprenant en outre un organe de recouvrement (8) recouvrant l'ensemble de l'organe de maintien d'IP (5, 5A, 5B).

7. Ensemble de protection de plaque d'imagerie (3) selon l'une des revendications 1 à 6, dans lequel l'organe de maintien d'IP (5, 5A, 5B) est un matériau laissant passer un rayonnement et est en papier ou en résine.

8. Ensemble d'imagerie médicale (1) comprenant :
l'ensemble de protection de plaque d'imagerie (3) selon l'une des revendications 1 à 7 ; et
une pluralité de plaques d'imagerie (2) logées dans l'ensemble de protection de plaque d'imagerie (3).
